# FASCICULE DE BREVET EUROPEEN

(11) **EP 2 257 230 B1**
(45) Date de publication et mention de la délivrance du brevet: **28.09.2011**
(21) Numéro de dépôt: 09723554.3
(22) Date de dépôt: 05.03.2009
(51) Int. Cl.: A61B 17/17, A61B 17/86, A61B 17/00, A61B 17/16, A61B 17/80

(54) **MATERIEL POUR LA REDUCTION D'UNE FRACTURE, EN PARTICULIER D'UNE FRACTURE SITUEE AU NIVEAU DES EPIPHYSES OSSEUSES**
VORRICHTUNG ZUR REDUKTION EINER FRAKTUR, IM BESONDEREN EINER FRAKTUR IM BEREICH DER EPIPHYSE
EQUIPMENT FOR REDUCING A FRACTURE, IN PARTICULAR A FRACTURE SITUATED IN THE AREA OF THE EPIPHYSES

(30) Priorité: 07.03.2008 FR 0801266
(43) Date de publication de la demande: 08.12.2010
(73) Titulaire: D.L.P., 44690 La Haye Fouassiere (FR)
(72) Inventeur: DEROUET, Guillaume, F-44800 Saint Herblain (FR); LARCHE, Grégoire, F-49300 Cholet (FR)
(74) Mandataire: Michelet, Alain
(86) Numéro de dépôt international: PCT/FR2009/050364
(87) Numéro de publication internationale: WO 2009/115741

(56) Documents cités:
- EP-A- 0 613 658
- WO-A-03/101320
- WO-A-2005/092224

## Description

La présente invention concerne un matériel, ou plus précisément un ensemble de matériels (ou kit) proposé aux chirurgiens pour réduire une fracture osseuse, en particulier une fracture située au niveau des épiphyses osseuses (radius distal, humérus proximal, fémur distal...

Il existe de nombreux systèmes du genre plaque(s) associée(s) à un ensemble de vis de fixation, proposés aux chirurgiens pour réduire une fracture osseuse.

En fonction du type de fracture en présence, pour obtenir une réduction optimale, il peut être intéressant d'incliner l'axe d'une ou de chaque vis de fixation, de manière appropriée, par rapport à la perpendiculaire de la plaque support.

Pour cela, et pour des fractures simples, courantes ou relativement courantes, certaines plaques comportent des vis de fixation de type « mono-axial », avec une orientation prédéfinie. Pour faciliter la mise en place de ces vis, il est connu d'utiliser un dispositif appelé bloc-canon (encore dénommé « targeting guide ou fast drilling guide), rapporté de manière amovible sur la plaque support, équipé d'une pluralité d'orifices traversants, destinés chacun à venir en regard de l'un des orifices de la plaque support et dont l'axe est incliné correctement en fonction de l'inclinaison souhaitée pour la vis correspondante.
Ce bloc-canon permet donc une configuration prédéterminée pour les vis de fixation associées ; il est utilisé, en association avec un canon de perçage, pour servir de guide au matériel de perçage des avant-trous d'implantation des vis (moteur chirurgical et forêt), et pour servir de guide aux vis elles-mêmes lors de leur mise en place.

Pour des fractures complexes, le chirurgien peut utiliser des plaques munies de vis de fixation dites « poly-axiales », c'est-à-dire dont l'angle d'implantation dans l'os peut être réglé selon un domaine d'inclinaison admissible prédéterminé.
En fonction du cas en présence, le chirurgien peut alors choisir l'inclinaison des vis de fixation qu'il juge la plus optimale.
Ces vis sont mises en place après réalisation d'un avant-trou de perçage dans l'os, au moyen du matériel de perçage, associé à un canon de guidage préalablement correctement orienté selon la direction de fixation choisie.

De plus, dans tous les cas, qu'il s'agisse d'un implant d'ostéosynthèse à technique « mono-axiale » ou « poly-axiale » il est intéressant d'obtenir un verrouillage de la vis de fixation, en fin de vissage dans l'os, pour optimiser la stabilité mécanique du montage d'ostéosynthèse obtenu et limiter les risques de démontage de celui-ci (migration des vis...) durant la phase de consolidation osseuse, après remise en charge de l'os.

Ainsi, en pratique, en fonction de la fracture qu'il doit réduire, le chirurgien fait un choix entre les systèmes de type mono-axial ou poly-axial à sa disposition, ce choix étant généralement réalisé au cours de l'opération chirurgicale, après réduction de la fracture.

Les techniques utilisant les systèmes de type « mono-axial » avec bloc-canon sont moins onéreux et plus rapides à mettre en oeuvre, cependant ils n'offrent aux chirurgiens pratiquement aucune latitude d'implantation des vis.

D'un autre côté, les systèmes de type « poly-axial » offrent au chirurgien un choix d'implantation des vis, mais ils sont plus onéreux et nécessitent un temps de mise en oeuvre plus important.

Le document WO 03101320 A décrit un matériel pour la réduction d'une fracture.

La présente invention vise à optimiser l'intervention du chirurgien, en particulier-pour les fractures situées au niveau des épiphyses osseuses, en lui proposant un ensemble de matériels qu'il pourra utiliser quel que soit le type de fracture en présence, ceci, au choix pour chaque vis de fixation, selon une technique de type poly-axial ou de type mono-axial.

Conformément à l'invention, le matériel correspondant comprend :
- une plaque support d'ostéosynthèse comprenant une face de dessous et une face de dessus, ladite face de dessous étant destinée à être positionnée contre le matériau osseux de réception ; cette plaque comprend une partie de corps allongée, prolongée par une partie de tête monobloc, ladite partie de corps comprenant une pluralité d'orifices traversants et ladite partie de tête comprenant une pluralité d'orifices traversants dont au moins certains autorisent l'accueil de vis ayant un caractère poly-axial, c'est-à-dire aptes à être implantées selon un domaine d'inclinaison prédéterminé admissible, ladite plaque comportant encore, au niveau de la zone de liaison entre sa partie de corps et sa partie de tête, au moins un orifice fileté complémentaire,
- un jeu de vis de fixation dans l'os, destinées à être insérées dans lesdits orifices traversants du corps de plaque, dites « vis de corps », pour fixer ledit corps de plaque à la surface de l'os, lesquelles vis de corps comportent une tête prolongée par un corps muni d'un filetage de fixation dans l'os,
- un jeu de vis de fixation dans l'os, destinées à être insérées dans lesdits orifices traversants de la tête de plaque, dites « vis de tête », pour fixer ladite tête de plaque à la surface de l'os,
- un dispositif de type bloc-canon destiné à être positionné sur la face de dessus de ladite tête de plaque, pour permettre un positionnement défini en inclinaison desdites vis de tête, lequel bloc-canon consiste en un bloc de matière comportant une pluralité d'orifices traversants, en nombre identique à ceux de ladite tête de plaque, chacun destiné à venir en correspondance avec l'un desdits orifices de ladite tête de plaque, lesquels orifices traversants sont adaptés pour servir de guide auxdites vis de tête, et lequel bloc-canon comprend encore au moins un orifice traversant destiné à être positionné dans le prolongement dudit orifice complémentaire de la tête de plaque, pour sa fixation amovible sur celle-ci au moyen d'une vis de fixation adaptée,
- des moyens de perçage d'orifices, du type moteur chirurgical associé à un jeu de forêts adaptés,
- un premier canon de perçage, adapté pour venir coopérer avec les orifices de ladite tête de plaque, pour permettre le perçage dans l'os d'un avant-trou de réception pour lesdites vis de tête, selon une technique poly-axiale, et
- un second canon de perçage, adapté pour venir coopérer avec les orifices dudit bloc-canon positionné sur ladite tête de plaque, pour permettre le perçage dans l'os d'un avant-trou de réception pour l'une desdites vis de tête, selon une technique mono-axiale.

De préférence le matériel conforme à l'invention comprend une plaque d'ostéosynthèse dont l'un au moins des orifices traversants de la partie de tête est équipé de moyens assurant le verrouillage de la vis de tête associée sur ladite plaque, en fin d'opération de vissage dans le matériau osseux de réception.

Selon un mode de réalisation avantageux, l'un au moins des orifices traversants de la partie de tête de la plaque d'ostéosynthèse comprend un logement ménagé pour accueillir et maintenir un écrou, ledit écrou étant bloqué en rotation dans son logement par rapport à ladite plaque support et ledit écrou comportant une surface de contact sphérique apte à coopérer avec une surface de contact sphérique complémentaire ménagée dans son logement d'accueil, pour lui conférer un degré de liberté dans ledit logement , selon un domaine d'inclinaison prédéterminé admissible, pour permettre une inclinaison de son axe par rapport à l'axe dudit logement de réception, ledit écrou ayant de ce fait un caractère « poly-axial ». En outre, l'une au moins des vis de tête comporte alors une tête prolongée par un corps muni - d'un filetage de fixation dans l'os, et - d'un filetage destiné à coopérer avec le filetage de l'écrou de plaque associé.

Dans le cadre de ce mode de réalisation avantageux :
- ledit premier canon de perçage est muni d'un embout fileté adapté pour venir se visser dans l'un des écrous de la tête de plaque, et
- ledit second canon de perçage est adapté pour venir coopérer avec l'un des orifices dudit bloc-canon, positionné en butée sur l'un desdits écrous de ladite tête de plaque.

Le premier canon de perçage comporte avantageusement une tête structurée et dimensionnée pour pouvoir traverser les orifices du bloc-canon, ceci avec un jeu lui autorisant un degré de liberté selon un domaine d'inclinaison, pour permettre la mise en oeuvre d'une technique poly-axiale des vis de tête associées, au travers desdits orifices du bloc-canon en place sur la plaque d'ostéosynthèse.

De préférence encore, le bloc-canon est muni d'un unique orifice traversant pour sa fixation sur la plaque d'ostéosynthèse et il comporte également, sur sa face de dessous destinée à venir en contact avec la tête de plaque, au moins un ergot ou une réservation destiné(e) à coopérer avec un ergot ou une réservation complémentaire ménagé(e) en correspondance sur la face de dessus de ladite tête de plaque, afin d'assurer le centrage correct dudit bloc-canon sur ladite tête de plaque.

Selon encore une autre particularité, le matériel proposé au chirurgien comprend un jeu de broches de positionnement ; en outre, le bloc-canon et la plaque d'ostéosynthèse (au niveau de sa partie de tête), comportent des orifices en correspondance pour le passage de ces broches de positionnement.

Le second canon de perçage (utilisé en association avec le bloc-canon) comporte avantageusement un système de graduation destiné à coopérer avec un repère ménagé sur le forêt de perçage associé, en vue de déterminer la profondeur de perçage réalisée, et de déterminer ainsi la longueur de vis adéquate à utiliser.

D'autre part, le matériel mis à disposition du chirurgien comporte encore avantageusement une jauge apte à déterminer la longueur de l'avant-trou traversant réalisé au travers de l'os percé, laquelle jauge comporte un corps cylindrique creux dont l'embout est adapté pour venir prendre appui sur la plaque d'ostéosynthèse, éventuellement au travers de l'un des orifices du bloc-canon, associé à un coulisseau dont l'une des extrémités est munie d'un système gradué et dont l'autre extrémité est munie d'un crochet de positionnement.

L'invention sera encore illustrée, sans être aucunement limitée, par la description suivante de différentes techniques opératoires particulières, utilisant l'ensemble de matériels conforme à l'invention, ceci en relation avec les dessins annexés dans lesquels :
- la figure 1 est une vue en perspective d'une plaque d'ostéosynthèse faisant partie du matériel proposé ;
- la figure 2 est une vue en perspective du bloc-canon faisant partie du matériel proposé ;
- la figure 3 montre l'association plaque/bloc-canon en cours de positionnement correct sur l'extrémité d'un radius, en association avec des broches de positionnement ;
- la figure 4 illustre le perçage d'un avant-trou dans l'extrémité du radius, pour le positionnement d'une vis de tête selon une technique mono-axiale utilisant le bloc-canon et un canon de perçage adapté ;
- la figure 5 est une vue selon un plan de coupe de la figure 4 passant par l'orifice du bloc-canon dans lequel est positionné le canon de perçage ;
- la figure 6 montre la mise en place d'une vis de tête au travers du bloc-canon, suite au perçage de l'avant-trou illustré figures 4 et 5 ;
- la figure 7 montre la jauge permettant de déterminer la longueur d'un avant-trou traversant réalisé dans l'os ;
- la figure 8 montre l'utilisation de la jauge illustrée figure 7, sur l'ensemble plaque/bloc-canon ;
- la figure 9 montre l'utilisation de l'autre canon de perçage d'avant-trou pour la mise en oeuvre de la technique de fixation poly-axiale ;
- la figure 10 est une vue selon un plan de coupe de la figure 9 passant par l'orifice de la plaque d'ostéosynthèse dans lequel est positionné le canon de perçage ;
- la figure 11 est une vue de la plaque d'ostéosynthèse convenablement montée sur le radius avec l'ensemble de ses vis de fixation ;
- la figure 12 est une vue isolée de la plaque, montrant l'implantation des différentes vis de tête ;
- la figure 13 est une vue en coupe illustrant une variante de réalisation dans laquelle le canon de perçage pour la mise en oeuvre de la technique poly-axiale peut être utilisé avec le bloc-canon en place sur la plaque d'ostéosynthèse ;
- la figure 14 est une vue en coupe dérivée de la figure 13 qui montre une autre angulation du canon de perçage, illustrant le caractère poly-axial de la technique, malgré la présence du bloc-canon ;
- les figures 15 et 16 sont des vues en perspective, respectivement par-dessus et par-dessous, d'une variante de plaque d'ostéosynthèse susceptible de faire partie du matériel proposé, associée à son bloc-canon.

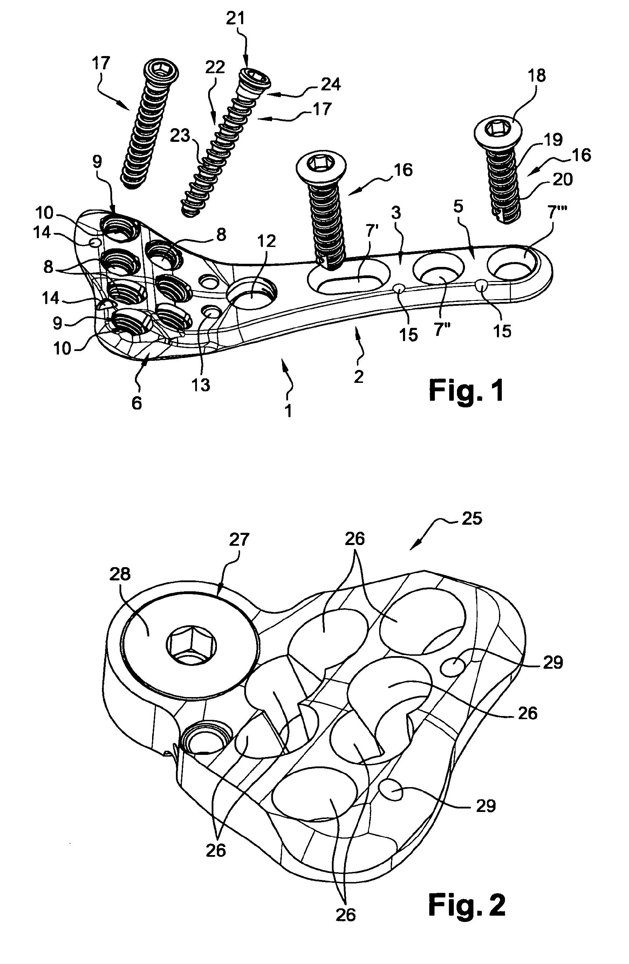

Tel qu'illustré sur les figures 1 à 12, le matériel conforme à l'invention proposé au chirurgien, pour la réduction d'une fracture de radius distal, comprend notamment une plaque d'ostéosynthèse, un jeu de vis diaphysaires, un jeu de vis épiphysaires, un bloc-canon, deux canons de perçage, un jeu de broches de positionnement et un matériel de perçage (moteur chirurgical et jeu de forêts adaptés).

La plaque d'ostéosynthèse 1 illustrée isolément sur la figure 1 comporte une face de dessous 2 destinée à venir en contact avec le matériau osseux et une face de dessus 3. Cette plaque 1 est constituée d'une partie de corps allongée 5 adaptée pour venir se positionner sur la diaphyse de l'os, et d'une partie de tête 6, monobloc, adaptée pour venir se positionner sur l'épiphyse osseuse.

La partie de corps 5 comprend une pluralité d'orifices alignés traversants 7', 7", 7"' (en l'occurrence trois), dont l'un 7' présente une forme allongée oblongue. La partie de tête 6 comprend quant à elle une pluralité d'orifices traversants 8 arrangés sur deux lignes sensiblement perpendiculaires à l'axe de la partie de corps 5. La ligne distale (d'extrémité) est composée d'un alignement de quatre orifices 8 alors que la ligne proximale est composée d'un alignement de trois orifices 8.

Chaque orifice de tête 8 comporte un logement 9 d'accueil d'un écrou « poly-axial » 10, par exemple tel que décrit dans les documents FR-A-2 832 308 et FR-07 02777.

Cet écrou 10 comporte un orifice central fileté et une surface de contact sphérique (non visible sur la figure 1) adaptée pour coopérer avec une surface sphérique complémentaire ménagée dans le logement d'accueil 9 (également non visible sur la figure 1) pour lui conférer son caractère poly-axial (c'est-à-dire pour lui conférer un degré de liberté en inclinaison) selon un domaine d'inclinaison prédéterminé admissible. Des moyens du type tenon/mortaise sont aussi prévus pour bloquer les écrous 10 en rotation dans leur logement d'accueil 9 (ces moyens n'apparaissent pas sur la figure 1).

Au niveau de la zone de liaison entre la partie de corps 5 et la partie de tête 6, la plaque 1 comporte un orifice complémentaire 12 muni d'un filetage.

En outre, sur la face de dessus de la partie de tête 6, juste devant l'orifice complémentaire 12, on remarque la présence de deux petites mortaises traversantes 13 en forme de réservations cylindriques.

L'extrémité distale de la partie de tête 6 comporte encore deux petits orifices traversants non filetés 14. Sur l'un des côtés de la partie de corps 5, on remarque également la présence de deux petits orifices traversants non filetés 15, destinés à recevoir temporairement, et si besoin, des broches qui pourront maintenir la plaque sur la diaphyse avant la mise en place des vis de fixation.

Le matériel selon l'invention comporte aussi un jeu de vis 16 pour la fixation sur l'os du corps de plaque 5 par l'intermédiaire des orifices traversants 7 (dites « vis de corps »), et un jeu de vis 17 pour la fixation sur l'os de la tête de plaque 6 par l'intermédiaire des orifices traversants 8 (dites « vis de tête »). Les vis de corps 16 sont chacune constituées d'une tête 18 prolongée par un corps 19 muni d'un filetage d'os 20. Les vis de tête 17 sont chacune constituées d'une tête 21 prolongée par un corps 22 muni d'un filetage d'os 23 et d'un filetage 24 destiné à coopérer avec le filetage de l'orifice de l'un des écrous 10.

D'autre part, l'ensemble de matériels selon l'invention comprend également un dispositif connu appelé bloc-canon 25, tel qu'illustré sur la figure 2. Ce bloc-canon 25 consiste en un bloc de matière ayant par exemple une épaisseur de l'ordre de 4 à 6 mm, dont la forme générale correspond approximativement à celle de la partie de tête 6 de la plaque d'ostéosynthèse 1, et qui est muni d'orifices traversants 26. Le nombre de ces orifices traversants 26 est identique à celui des orifices 8 de la partie de tête 6 de la plaque 1 (à savoir sept) et ces orifices 26 sont ménagés pour venir chacun en correspondance avec l'un de ces orifices de tête 8, lorsque ledit bloc-canon 25 est monté sur ladite plaque 1.

Le bloc-canon 25 comporte encore un orifice 27 destiné à être positionné en regard de l'orifice complémentaire fileté 12 de la plaque 1, pour le positionnement d'une vis 28 permettant sa fixation amovible sur ladite plaque 1. Le positionnement correct.centré du bloc-canon 25 sur la tête de plaque 6 est obtenu au moyen d'ergots ou de pions saillants monoblocs cylindriques (non visibles sur la figure 2) qui équipent sa face de dessous et qui sont destinés à pénétrer dans les réservations complémentaires 13 précitées de la tête de plaque 6.

Les orifices traversants 26 du bloc-canon 25 sont cylindriques et non filetés ; ils sont destinés chacun à servir de guide pour le positionnement précis en orientation des vis de tête 17.
L'axe de chacun des orifices 26 est particulier, orienté selon une direction prédéterminée correspondant à la direction précise optimale pour la vis de tête 17 destinée à être positionnée dans l'orifice de tête 8 en correspondance, ceci pour les fractures courantes, rencontrées relativement fréquemment.

Le bloc-canon 25 comporte encore deux petits orifices traversants 29 destinés à venir en correspondance avec les orifices d'extrémités 14 de la tête de plaque 6, adaptés, comme on le verra ci-après, pour le passage de broches de positionnement.

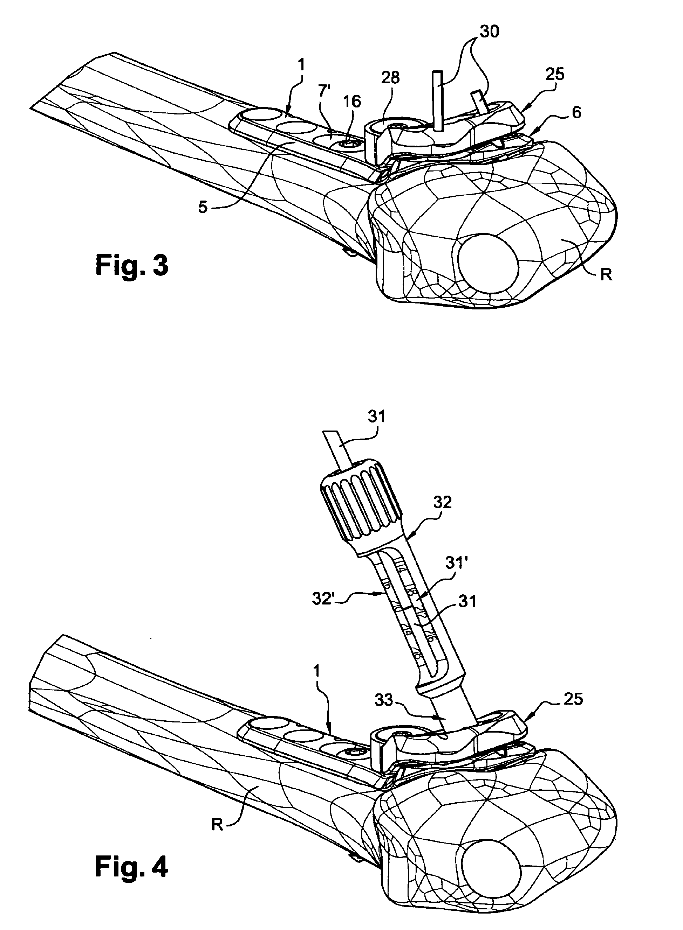

Pour la réduction d'une fracture de radius distal de complexité moyenne, le chirurgien pose le bloc-canon 25 sur la tête de plaque 6 au moyen de la vis de fixation 28 ; les ensembles précités de tenons/réservations associés assurent le centrage et le positionnement correct des deux éléments l'un par rapport à l'autre.
Ensuite, il pose du mieux possible la plaque d'ostéosynthèse 1 sur le radius R et il fixe sa partie de corps 5 sur la structure osseuse au moyen d'une vis de corps 16 dans l'orifice oblong 7' (figure 3).
Le chirurgien contrôle alors le positionnement correct de la plaque en enfonçant deux broches de positionnement 30 dans les couples d'orifices 14-29 du bloc-canon 25 et de la tête de plaque 6, ainsi qu'au travers de l'os R, de manière à s'assurer qu'elles ne débouchent pas dans l'articulation du radius. Il s'agit là d'une sécurité permettant d'éviter que les vis de tête 17 posées ultérieurement débouchent dans l'articulation du radius.
Le contrôle correspondant sur les broches de positionnement 30 est réalisé par radiologie ou amplificateur de brillance.
Le cas échéant, le positionnement de la plaque 1 est modifié longitudinalement par léger desserrage de la vis de corps 16 suivi d'un nouveau contrôle.
Une fois la plaque 1 jugée correctement positionnée, la vis de corps 16 est serrée.

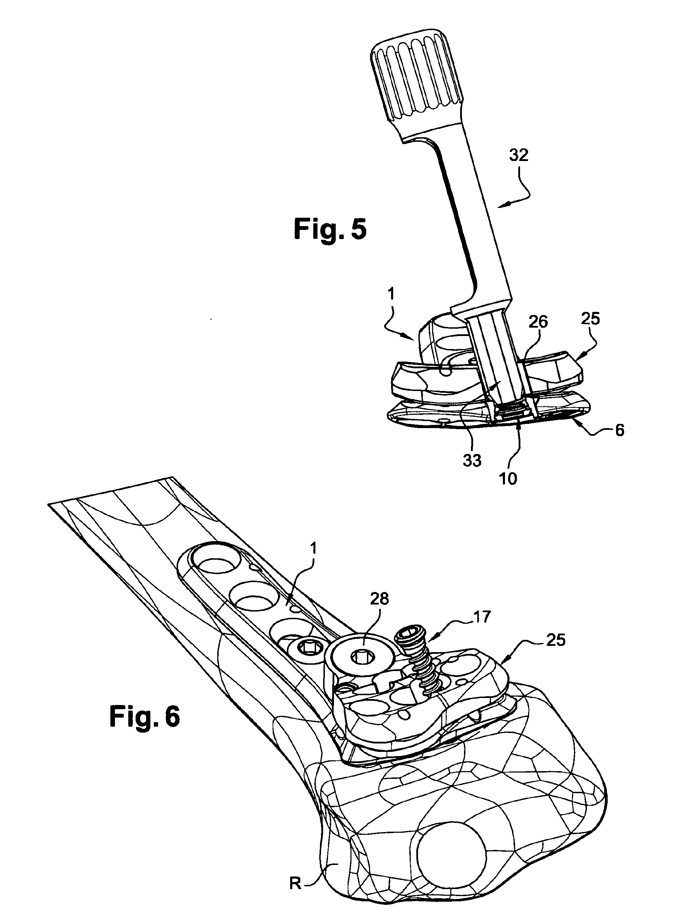

Le chirurgien a ainsi la possibilité de poser certaines au moins des vis de tête 17 selon une technique « mono-axiale » par l'intermédiaire du bloc-canon 25.
Pour chacune des vis 17 correspondantes, tel qu'illustré sur les figures 4 et 5, on perce un avant-trou dans l'os au moyen d'un moteur chirurgical (non représenté) équipé d'un forêt 31, en association avec un canon de perçage 32. L'extrémité cylindrique 33 du canon de perçage 32 est guidée par l'un des orifices 26 du bloc-canon 25 et elle vient se positionner en butée sur l'écrou 10 en correspondance logé dans la tête de plaque 6. La tête cylindrique 33 du canon de perçage 32 a un diamètre correspondant, au jeu près, au diamètre des orifices 26 du bloc-canon 25.
L'avant-trou correspondant est réalisé jusqu'à ce que la tête du forêt 31 atteigne la corticale opposée de l'os (pour obtenir un bon accrochage des vis 17 avec une prise la plus longue possible).
Un repère 31' sur le forêt 31, associé à une graduation 32' ménagée sur le canon de perçage 32 permettent de connaître avec précision la longueur de vis de tête 17 qui devra ensuite être utilisée.

Après enlèvement du forêt 31 et du canon de perçage 32, la vis correspondante 17 est mise en place (figure 6) au travers de l'orifice 26 associé du bloc-canon 25. En fin de vissage, cette vis 17 est mise en compression sur la plaque 1 et verrouillée par la présence de l'écrou 10 associé.

L'opération est recommencée pour toutes les vis de tête 17 que le chirurgien souhaite implanter selon cette technique « mono-axiale ».

Cette mise en oeuvre facilite sensiblement le travail du chirurgien et limite grandement le temps opératoire de l'ostéosynthèse.

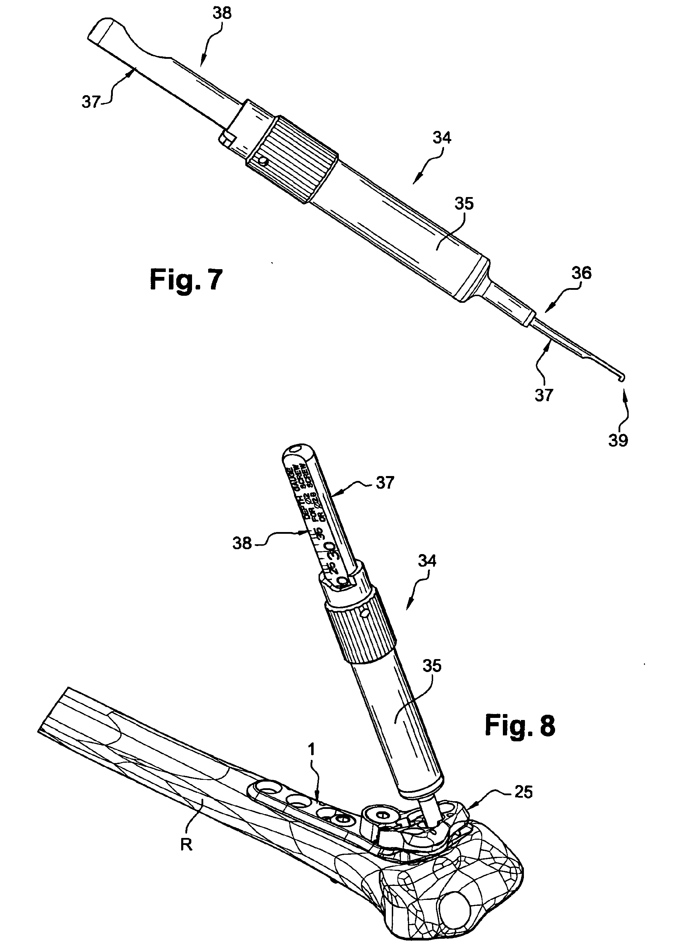

Si le chirurgien traverse complètement le radius R lors du perçage de l'avant-trou de vis, le matériel à sa disposition comprend une jauge 34, illustrée isolément sur la figure 7, adaptée pour déterminer la dimension des vis 17 à utiliser.

Cette jauge 34 comporte un corps 35 de forme générale cylindrique creuse, dont la tête 36 est agencée pour passer au travers de l'orifice 26 correspondant du bloc-canon 25 et pour venir prendre appui sur la face de dessus 3 de la plaque d'ostéosynthèse 1. Cette jauge 34 comporte encore un coulisseau 37, logé dans le corps creux 35, dont la partie supérieure 38 est graduée et dont l'extrémité inférieure est constituée d'une tige munie d'un crochet d'extrémité 39. On comprend bien qu'une fois le corps de jauge 35 en appui sur la plaque 1, le coulisseau 37 peut être manoeuvré de sorte à venir accrocher la corticale opposée de l'os, au travers de l'avant-trou réalisé, cet accrochage permettant de déterminer la profondeur exacte de l'avant-trou au moyen de la graduation 38, en regard de l'extrémité supérieure du corps creux 35, pour définir la longueur de vis adéquate (c'est-à-dire une vis dont l'extrémité atteindra la corticale opposée de l'os). La figure 8 illustre l'utilisation de cette jauge 34 sur la plaque 1 équipée du bloc-canon 25.

Lorsque le chirurgien a mis en place toutes les vis 17 qu'il souhaitait poser en technique « mono-axiale », il enlève le bloc-canon 25 (simplement par dévissage de la vis 28). Il peut alors poser les vis de tête restantes 17 selon une technique « poly-axiale ».

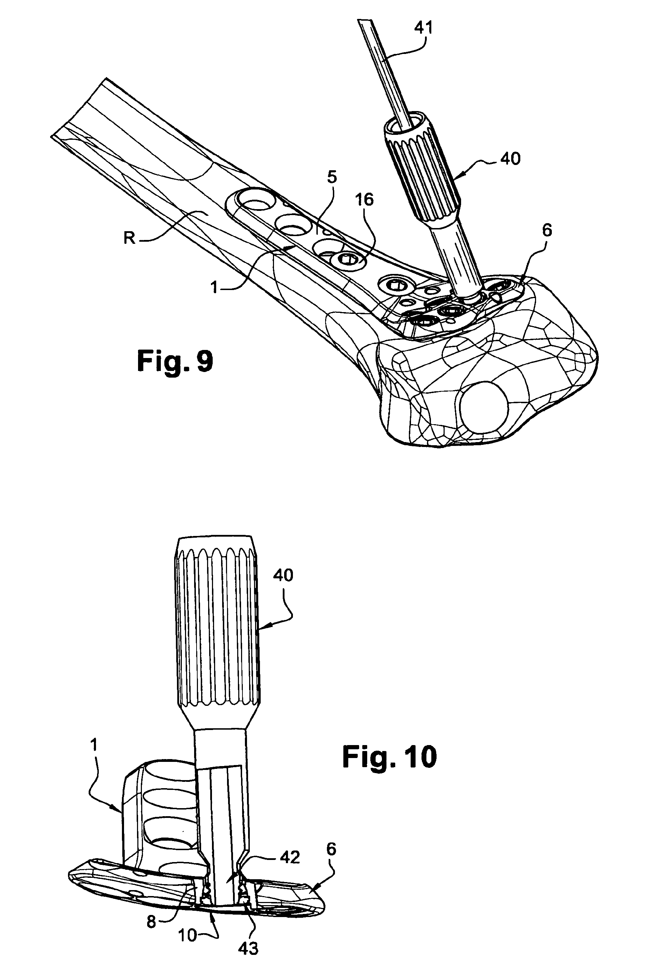

Il réalise pour cela des avant-trous en utilisant un canon de perçage 40 (figures 9 et 10) associé à un moteur chirurgical (non représenté) muni d'un forêt 41 adapté (visible uniquement sur la figure 9). La tête 42 du canon de perçage 40 est munie d'un filetage 43 et elle est vissée dans l'écrou 10 associé à l'orifice de tête 8 correspondant ; le chirurgien règle l'angulation du canon 40 pour percer convenablement l'avant-trou selon la direction souhaitée ; il perce l'avant-trou jusqu'à la corticale opposée de l'os et il utilise la jauge 34 précitée, illustrée sur la figure 7, pour déterminer la longueur de vis adéquate à utiliser.

Le chirurgien peut terminer la pose des vis de tête 17 en répétant cette succession d'opérations.

La fixation de la plaque d'ostéosynthèse 1 est terminée par mise en place des vis de corps restantes 16. On notera ici que l'orifice complémentaire 12 de la plaque d'ostéosynthèse 1 est avantageusement utilisé pour fixer une vis de corps 16 identique aux autres.

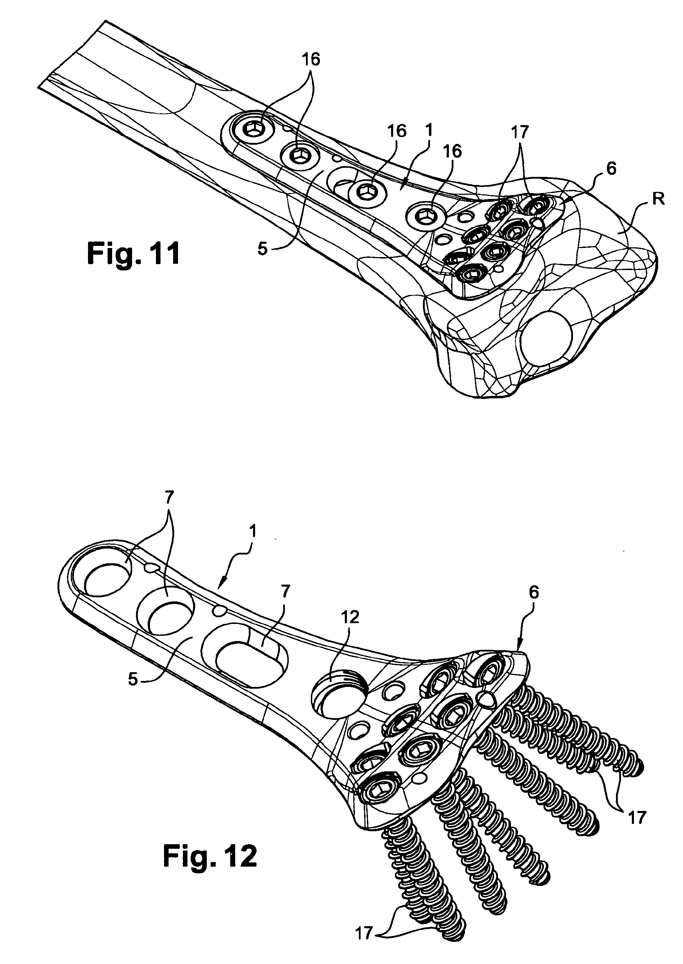

On obtient une plaque d'ostéosynthèse 1 posée et fixée sur le radius R tel qu'illustré sur la figure 11.
La figure 12 illustre une configuration possible d'angulation des différentes vis de tête 17.

En fonction du type de fracture en présence, l'ensemble de matériels conforme à l'invention peut aussi être utilisé par le chirurgien pour poser toutes les vis de tête 17 en technique mono-axiale, en utilisant uniquement le bloc-canon 25 (en particulier pour les fractures simples), ou pour poser l'ensemble des vis de tête 17 en technique poly-axiale, par utilisation uniquement du canon de perçage 40 (en particulier pour les fractures très complexes).

Lorsqu'il souhaite utiliser les deux techniques, le chirurgien commence de préférence par la technique mono-axiale de manière à utiliser un bloc-canon 25 pré positionné sur la plaque d'ostéosynthèse 1 et de manière à utiliser la technique poly-axiale sur une plaque d'ostéosynthèse 1 déjà sécurisée en position sur l'os.

Dans le mode de réalisation décrit ci-dessus, le canon de perçage 40 est utilisé après enlèvement du bloc-canon 25.

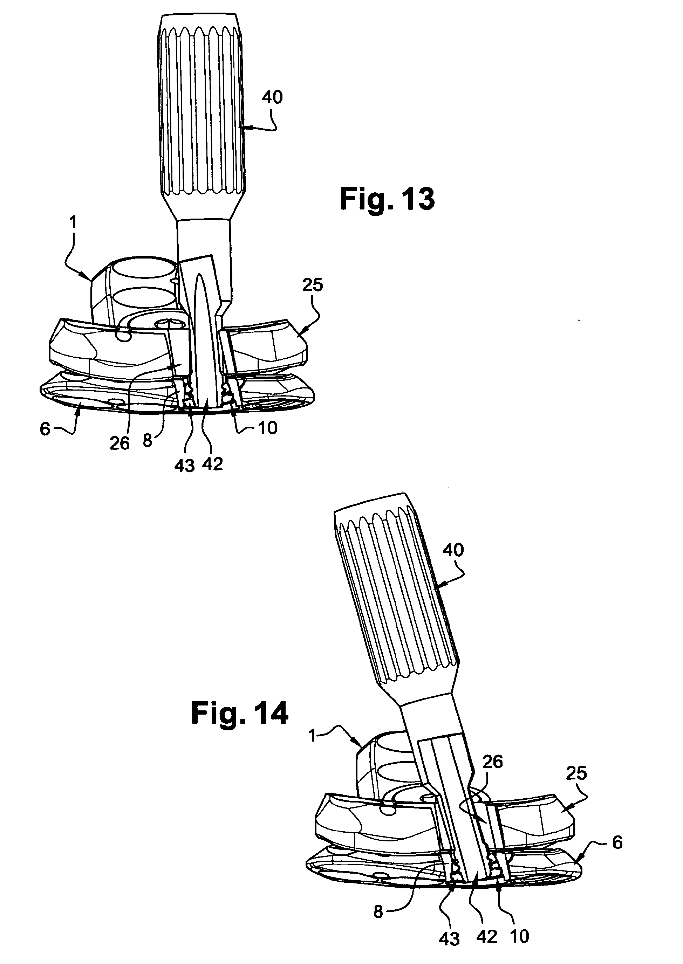

Selon une variante de réalisation illustrée sur les figures 13 et 14, le bloc-canon 25 et le canon de perçage 40 sont structurés pour permettre l'implantation des vis de tête 17 en technique poly-axiale tout en conservant le bloc-canon 25 en position sur la plaque d'ostéosynthèse 1.

Comme illustré sur les figures 13 et 14, la tête 42 du canon de perçage 40 est structurée et dimensionnée pour permettre son passage au travers des orifices 26 du bloc-canon 25, et ceci tout en conservant une certaine plage d'angulation possible par rapport à l'axe des orifices superposés 8 et 26. Cette plage d'angulation possible correspond avantageusement à celle qui est admise par les écrous 10 dans leurs logements d'accueil 9. La présence du bloc-canon 25 permet alors aux contours des orifices 26 de constituer une butée mécanique empêchant le chirurgien d'aller au-delà de la plage d'angulation admissible.

Les figures 13 et 14 montrent deux angulations différentes du canon de perçage 40 pour illustrer ce principe.

Le fait de pouvoir travailler en technique poly-axiale tout en conservant le bloc-canon 25 en position sur la plaque d'ostéosynthèse facilite et accélère le travail du chirurgien. En effet, il peut utiliser le bloc-canon 25 pour commencer à travailler en technique mono-axiale (simple et rapide), puis, en cours d'opération, en fonction des difficultés rencontrées ou de la complexité détectée de la fracture, passer sans problème, très rapidement, à une technique d'implantation poly-axiale d'une ou de plusieurs vis, sans avoir besoin d'enlever ledit bloc-canon 25.

Après mise en oeuvre de la technique poly-axiale pour une ou plusieurs vis, le chirurgien peut alors revenir tout aussi simplement à une technique d'implantation mono-axiale, le bloc-canon 25 étant déjà en place.

La présente invention peut s'appliquer à la réduction d'autres fractures épiphysaires que celles du radius distal, par exemple les fractures d'humérus proximal, de fémur distal ...

La structure de la plaque d'ostéosynthèse est adaptée à l'application envisagée. Sa forme générale sera en particulier fonction de la configuration physique spatiale de la zone de fracture que l'on cherche à réduire. Selon le cas, les orifices de tête 8 peuvent ne pas tous être équipés de moyens d'accueil de vis ayant un caractère poly-axial.

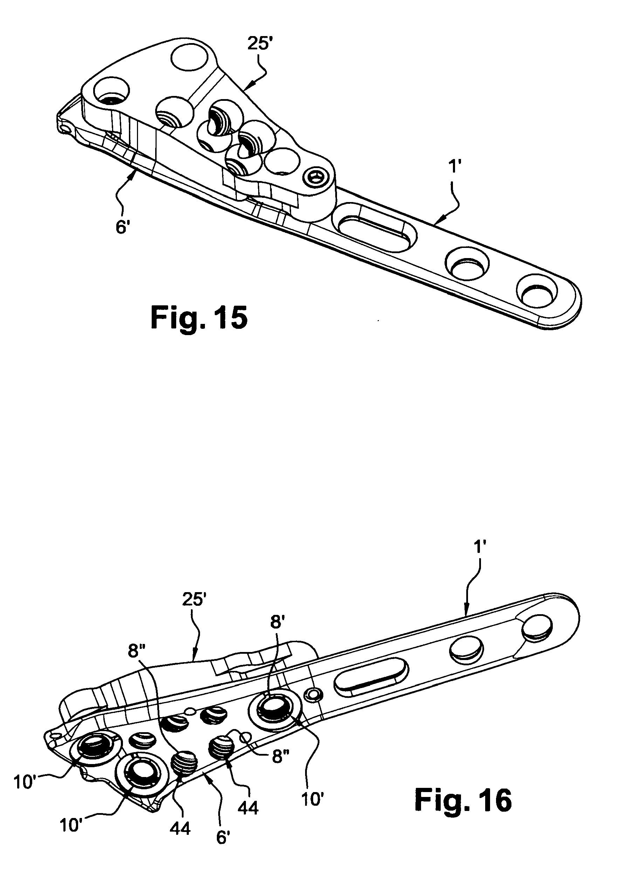

A titre d'exemple, les figures 15 et 16 montrent une plaque épiphysaire humérale 1' équipée de son bloc-canon amovible 25' et dont la tête 6' est munie d'orifices équipés, pour certains 8', d'écrous 10' pour la réception de vis à caractère poly-axial, et pour les autres 8" d'un simple filetage intérieur 44 pour la réception de vis à caractère mono-axial.

## Revendications

1. Matériel pour la réduction d'une fracture, en particulier d'une fracture située au niveau des épiphyses osseuses, ledit matériel comprenant en combinaison :
- une plaque support d'ostéosynthèse (1, 1') comprenant une face de dessous (2) et une face de dessus (3), ladite face de dessous (2) étant destinée à être positionnée contre le matériau osseux de réception (R), laquelle plaque (1, 1') comprend une partie de corps allongée (5), prolongée par une partie de tête (6, 6') monobloc, ladite partie de corps (5) comprenant une pluralité d'orifices traversants (7) et ladite partie de tête (6, 6') comprenant une pluralité d'orifices traversants (8 ; 8', 8"), dont certains au moins autorisent l'accueil de vis ayant un caractère poly-axial, c'est-à-dire aptes à être implantées selon un domaine d'inclinaison prédéterminé admissible, ladite plaque (1, 1') comportant encore, au niveau de la zone de liaison entre la partie de corps (5) et la partie de tête (6, 6'), au moins un orifice fileté complémentaire (12),
- un jeu de vis (16) de fixation dans l'os, destinées à être insérées dans lesdits orifices traversants (7) du corps de plaque (5), dites « vis de corps », pour fixer ledit corps de plaque (5) à la surface de l'os, lesquelles vis de corps (16) comportent une tête (18) prolongée par un corps (19) muni d'un filetage (20) de fixation dans l'os,
- un jeu de vis (17) de fixation dans l'os, destinées à être insérées dans lesdits orifices traversants (8 ; 8', 8") de ladite partie de tête (6, 6'), dites « vis de tête », pour fixe ladite partie de tête (6, 6') à la surface de l'os,
- un dispositif de type bloc-canon (25, 25') destiné à être positionné sur la face de dessus (3) de ladite partie de tête (6, 6'), pour permettre un positionnement défini en inclinaison desdites vis de tête (17), lequel bloc-canon (25, 25') consiste en un bloc de matière comportant une pluralité d'orifices traversants (26), en nombre identique à ceux (8 ; 8',_8") de ladite partie de tête (6, 6'), chacun destiné à venir en correspondance avec l'un desdits orifices (8 ; 8', 8"), lesquels orifices traversants (26) dudit bloc-canon sont adaptés pour servir de guide auxdites vis de tête (17), et lequel bloc-canon (25, 25') comprend encore au moins un orifice traversant (27) destiné à être positionné dans le prolongement dudit orifice complémentaire (12) de ladite partie de tête (6, 6'), pour sa fixation amovible sur celle-ci au moyen d'une vis de fixation (28) adaptée,
- des moyens de perçage d'orifices, du type moteur chirurgical associé à un jeu de forêts adaptés (31, 41), et
- un second canon de perçage (32), adapté pour venir coopérer avec les orifices (26) dudit bloc-canon (25, 25') positionné sur ladite partie de tête (6, 6'), pour permettre le perçage dans l'os d'un avant-trou de réception pour lesdites vis de tête (17), selon une technique mono-axial,
**caractérisé en ce que** ledit matériel comprend en plus :
- un premier canon de perçage (40), adapté pour venir coopérer avec les orifices (8 ; 8', 8") de ladite partie de tête (6, 6'), pour permettre le perçage dans l'os d'un avant-trou de réception pour lesdites vis de tête (17), selon une technique poly-axiale.

2. Matériel selon la revendication 1, **caractérisé en ce qu'**il comprend une plaque d'ostéosynthèse (1) dont l'un au moins des orifices traversants (8 ; 8', 8") de la partie de tête (6, 6') est équipé de moyens assurant le verrouillage de la vis de tête associée (17) sur ladite plaque (1, 1'), en fin d'opération de vissage dans le matériau osseux de réception.

3. Matériel selon la revendication 2, **caractérisé en ce que** l'un au moins des orifices traversants (8 ; 8', 8") de la partie de tête (6, 6') de la plaque d'ostéosynthèse (1, 1') comprend un logement (9) ménagé pour accueillir et maintenir un écrou (10, 10'), ledit écrou (10, 10') étant bloqué en rotation dans son logement (9) par rapport à ladite plaque support (1, 1') et ledit écrou (10, 10') comportant une surface de contact sphérique apte à coopérer avec une surface de contact sphérique complémentaire ménagée dans son logement d'accueil (9), pour lui conférer un degré de liberté dans ledit logement (9), selon un domaine d'inclinaison prédéterminé admissible, pour permettre une inclinaison de son axe par rapport à l'axe dudit logement (9) de réception, ledit écrou (10, 10') ayant de ce fait un caractère poly-axial, et **en ce que** l'une au moins des vis de tête (17) comporte une tête (21) prolongée par un corps (22) muni - d'un filetage (23) de fixation dans l'os, et - d'un filetage (24) destiné à coopérer avec le filetage de l'écrou de plaque (10) associé.

4. Matériel selon la revendication 3, **caractérisé en ce que**:
- ledit premier canon de perçage (40) est muni d'un embout fileté (42, 43) adapté pour venir se visser dans l'un des écrous (10, 10') de la partie de tête (6, 6'), et
- ledit second canon de perçage (32) est adapté pour venir coopérer avec l'un des orifices (26) dudit bloc-canon (25, 25'), positionné en butée sur l'un desdits écrous (10) de ladite partie de tête (6, 6').

5. Matériel selon l'une quelconque des revendications 1 à 4, **caractérisé en ce que** ledit premier canon de perçage (40) comporte une tête (42) structurée et dimensionnées pour pouvoir traverser les orifices (26) du bloc-canon (25, 25') ceci avec un jeu lui autorisant un degré de liberté selon un domaine d'inclinaison, pour permettre la mise en oeuvre d'une technique poly-axiale de la vis de tête associée au travers dudit orifice (26) du bloc-canon (25, 25') en place sur la plaque d'ostéosynthèse (1, 1').

6. Matériel selon l'une quelconque des revendications 1 à 5, **caractérisé en ce qu'**il comprend un bloc-canon (25, 25') muni d'un unique orifice traversant (27) pour sa fixation sur la plaque d'ostéosynthèse (1, 1') et muni, sur sa face de dessous destinée à venir en contact avec la partie de tête (6, 6'), d'au moins un ergot ou une réservation destiné(e) à coopérer avec un ergot ou une réservation complémentaire (13) ménagé(e) en correspondance sur la face de dessus (3) de ladite partie de tête (6, 6').

7. Matériel selon l'une quelconque des revendications 1 à 6, **caractérisé en ce qu'**il comprend un jeu de broches de positionnement (30), et **en ce que** le bloc-canon (25, 25') et la partie de tête (6, 6') comportent des orifices en correspondance (14, 29) pour le passage desdites broches de positionnement (30).

8. Matériel selon l'une quelconque des revendications 1 à 7, **caractérisé en ce que** le second canon de perçage (32) comporte un système de graduation (32') destiné à coopérer avec un repère (31') ménagé sur le forêt de perçage associé (31), en vue de déterminer la profondeur de perçage réalisée, et de déterminer ainsi la longueur de vis (17) adéquate à utiliser.

9. Matériel selon l'une quelconque des revendications 1 à 8, **caractérisé en ce qu'**il comprend une jauge (34) apte à déterminer la longueur de l'avant-trou traversant réalisé au travers de l'os percé, laquelle jauge (34) comporte un corps cylindrique creux (35) dont l'embout (36) est adapté pour venir prendre appui sur la plaque d'ostéosynthèse (1, 1'), éventuellement au travers de l'un des orifices (26) du bloc-canon (25, 25'), associé à un coulisseau (37) dont l'une des extrémités est munie d'un système gradué (38) et dont l'autre extrémité est munie d'un crochet de positionnement (39).

## Claims

1. A device for reducing a fracture, in particular a fracture at the bone epiphyses, said device comprising, in combination :
- an osteosynthesis supporting plate (1, 1') including a bottom face (2) and a top face (3), said bottom face (2) intended for being located against the receiving bone material (R), which plate (1, 1') comprises an elongated body part (5), prolongated by a one-piece head part (6, 6'), said body part (5) including a plurality of through-orifices (7) and said head part (6, 6') including a plurality of through-orifices (8 ; 8', 8"), at least some of them enabling to accommodate poly-axial type screws, i.e. capable of being implanted along an admissible predetermined tilting range, said plate (1, 1') still comprising, at the linking zone between the body part (5) and the head part (6, 6'), at least one complementary threaded orifice (12),
- a set of screws (16) for fastening into the bone, intended for being inserted into said through-orifices (7) of the plate body (5), so-called "body screws", for attaching said plate body (5) to the surface of the bone, which body screws (16) comprise a head (18) prolongated by a body (19) fitted with a thread (20) for fastening into the bone,
- a set of screws (17) for fastening into the bone, intended for being inserted into said through-orifices (8 ; 8', 8") of said head part (6, 6'), so-called "head screws", for attaching said head part (6, 6') to the surface of the bone,
- a targeting guide type device (25, 25') intended for being positioned on the top face (3) of said head part (6, 6'), to enable set tilting positioning of said head screws (17), which targeting guide (25, 25') consists in a piece of material comprising a plurality of through-orifices (26), identical in number to those (8 ; 8', 8") of said head part (6, 6'), each intended for matching one of said orifices (8 ; 8', 8"), which through-orifices (26) of said targeting guide are adapted for acting as a guide for said head screws (17), and which targeting guide (25, 25') still comprises at least one through-orifice (27) intended for being positioned in the extension of said complementary orifice (12) of the head part (6, 6'), for its removable fixture thereon by means of an adapted fastening screw (28),
- means for drilling orifices, of surgical motor type associated with a set of suitable drill bits (31, 41), and
- a second drilling guide (32), adapted for co-operating with the orifices (26) of said targeting guide (25, 25') positioned on said head part (6, 6'), to enable drilling into the bone a drill-hole for receiving said head screws (17), according a mono-axial technique, **characterised in that** said device further comprises:
- a first drilling guide (40), adapted for co-operating with the orifices (8 ; 8", 8") of said head part (6, 6'), to enable drilling into the bone a drill-hole for receiving said head screws (17), according a poly-axial technique.

2. A device according to claim 1, **characterised in that** it comprises an osteosynthesis plate (1) whereof at least one the through-orifices (8 ; 8', 8") of the head part (6, 6') is fitted with means for locking the associated head screw (17) on said plate (1, 1'), upon completion of the screwing operation into the receiving bone material.

3. A device according to claim 2, **characterised in that** at least one of the through-orifices (8 ; 8', 8") of the head part (6, 6') of the osteosynthesis plate (1, 1') comprises a housing (9) provided for accommodating and holding a nut (10, 10'), said nut (10, 10') being blocked into rotation in its housing (9) relative to said supporting plate (1, 1') and said nut (10, 10') comprising a spherical contact surface capable of co-operating with a complementary spherical contact surface provided in its reception housing (9), to confer said nut (10, 10') a degree of freedom in said housing (9), along an admissible predetermined tilting range, to enable tilting of its axis relative to the axis of said reception housing (9), said nut (10, 10') hence being of "poly-axial" type, and **in that** at least one of these head screws (17) compose a head (21) prolongated by a body (22) fitted - with a thread (23) for fastening into the bone, and - with a thread (24) intended for co-operating with the thread of the related plate nut (10).

4. A device according to claim 3, **characterised in that**:
- said first drilling guide (40) is fitted with a threaded insert (42, 43) adapted for being screwed in one of the nuts (10, 10') of the head part (6, 6'), and
- said second drilling guide (32) is adapted for co-operating with one of the orifices (26) of said targeting guide (25, 25'), abutting against one of said nuts (10) of said head part (6, 6').

5. A device according to any of the claims 1 to 4, **characterised in that** the first drilling guide (40) comprises a head (42) shaped and sized to come through the through-orifices (26) of the targeting guide (25, 25'), that with a clearance allowing it a degree of freedom according a tilting range, to permit the implementation of the poly-axial technique of the associated head screw through said orifice (26) of the targeting guide (25, 25') arranged onto the osteosynthesis plate (1, 1').

6. A device according to any of the claims 1 to 5, **characterised in that** it comprises a targeting guide (25, 25') fitted with a single through-orifice (27) for the attachment thereof on the osteosynthesis plate (1, 1') and fitted, on its bottom face intended for contacting the head part (6, 6'), with at least one toe or one recess intended for co-operating with one complementary toe or recess (13) provided to match on the top face (3) of said head part (6, 6').

7. A device according to any of the claims 1 to 6, **characterised in that** it comprises a set of positioning pegs (30), and **in that** the targeting guide (25, 25') and the head part (6, 6') comprise matching orifices (14, 29) for letting through said positioning pegs (30).

8. A device according to any of the claims 1 to 7, **characterised in that** the second drilling guide (32) comprises a graduation system (32') intended for co-operating with a mark (31 provided on the related drill bit (31), so as to determine the drilling depth performed, and thus to determine the suitable screw length (17) to be used.

9. A device according to any of the claims 1 to 8, **characterised in that** it comprises a gauge (34) capable of determining the length of the through drill-hole provided through the drilled bone, which gauge (34) comprises a hollow cylindrical body (35) whereof the insert (36) is adapted for resting on the osteosynthesis plate (1, 1'), optionally through one of the orifices (26) of the targeting guide (25, 25'), associated with a ram (37) whereof one of the ends is fitted with a graduated system (38) and whereof the other end is fitted with a positioning hook (39).

## Patentansprüche

1. Ausrüstung zur Reduktion einher Fraktur, insbesondere einer Fraktur, die sich an den Epiphysen bzw. Knochenenden befindet, wobei die Ausrüstung Folgendes in Kombination umfasst;
- eine Osteosynthese-Trägerplatte (1, 1'), die eine Unterseite (2) und eine Oberseite (3) umfasst, wobei die Unterseite (2) dazu gedacht ist, an dem Aufnahmeknochenmaterial (R) positioniert zu werden, wobei die Platte (1, 1') einen länglichen Körperteil (5) umfasst, der sich in einem einstückigen Kopfteil (6, 6') verlängert, wobei der Körperteil (5) eine Vielzahl von durchgehenden Öffnungen (7) umfasst und der Kopfteil (6, 6') eine Vielzahl von durchgehenden Öffnungen (8; 8', 8") umfasst, von denen mindestens einige die Aufnahme von mehrachsig beschaffenden Schrauben ermöglichen, d.h. die dazu geeignet sind, gemäß einem zulässigen vorherbestimmten Neigungsbereich implantiert zu werden, wobei die Platte (1, 1') zudem an dem Verbindungsbereich zwischen dem Körperteil (5) und dem Kopfteil (6, 6') mindestens eine komplementäre Gewindeöffnung (12) umfasst,
- einen Satz von Schrauben (16) zum Befestigen im Knochen, die dazu gedacht sind, in die durchgehenden Öffnungen (7) des Plattenkörpers (5) eingefügt zu werden, so genannte "Körpersclirauben", um den Plattenkörper (5) an der Oberfläche des Knochens zu befestigen, wobei die Körperschrauben (16) einen Kopf (18) umfassen, der sich in einem Körper (19) verlängert, der mit einem Gewinde (20) zur Befestigung im Knochen versehen ist,
- einen Satz Schrauben (17) zum Befestigen im Knochen, die dazu gedacht sind, in die durchgehenden Öffnungen (8; 8', 8") des Kopfteils (6, 6') eingefügt zu werden, so genannte "Kopfschrauben", um den Kopfteil (6, 6') an der Oberfläche des Knochens zu befestigen,
- eine Vorrichtung nach Art eines Zielbügels (25, 25'), die dazu gedacht ist, auf der Oberseite (3) des Kapfteils (6, 6') positioniert su werden, um eine neigungsmäßig definierte Positionierung der Kopfschrauben (17) zu ermöglichen, wobei der Zielbügel (25, 25') aus einem Materialblock besteht, der eine Vielzahl von durchgehenden Öffnungen (26) umfaßt, und zwar in der gleichen Anzahl wie diejenigen (8; 8', 8") des Kopfteils (6, 6"), die jeweils dazu gedacht sind, einer der Öffnungen (8; 8', 8") zu entsprechen, wobei die durchgehenden Öffnungen (26) des Zielbügels dazu geeignet sind, um als Führung für die Kopfschrauben (17) zu dienen, und wobei der Zielbügel (25, 25') zudem mindestens eine durchgehende Öffnung (27) umfasst, die dazu gedacht ist, in der Verlängerung der ergänzenden Öffnung (12) des Kopfteils (6, 6') für seine abnehmbare Befestigung daran mittels einer geeigneten Befestigungsschraube (28) positioniert zu werden,
- Mittel zum Bohren von Öffnungen, nach Art eines chirurgischen Motors, der mit einem Satz von geeigneten Bohrern (31, 41) verknüpft ist, und
- eine zweite Bohrbuchse (32), die dazu geeignet ist, mit den Öffnungen (26) des Zielbügels (25, 25') zusammenzuwirken, der auf dem Kopfteil (6, 6') positioniert ist, um es zu ermöglichen, im Knochen ein Aufnahme-Vorbohrloch für die Kopfschrauben (17) gemäß einer einachsigen Technik zu bohren,
**dadurch gekennzeichnet, dass** die Ausrüstung zudem Folgendes umfasst:
- eine erste Bohrbuchse (40), die dazu geeignet ist, um mit den Öffnungen (8; 8', 8") des Kopfteils (6, 6') zusammenzuwirken, um es zu ermöglichen, im Knochen ein Aufnahme-Vorbohrloch für die Kopfschrauben (17) gemäß einer mehrachsigen Technik zu bohren.

2. Ausrüstung nach Anspruch 1, **dadurch gekennzeichnet, dass** sie eine Osteosynthese-Platte (1) umfasst, wobei mindestens eine der durchgehenden Öffnungen (8; 8', 8") des Kopfteils (6, 6') mit Mitteln ausgestattet ist, die das Verriegeln der dazugehörigen Kopfschraube (17) auf der Platte (1, 1') am Ende des Schraubvorgangs in dem Aufnahmeknochenmaterial sicherstellen.

3. Ausrüstung nach Anspruch 2, **dadurch gekennzeichnet, dass** mindestens eine der durchgehenden Öffnungen (8; 8', 8") des Kopfteils (6, 6') der Osteosynthese-Platte (1, 1') eine Mulde (9) umfasst, die eingerichtet ist, um eine Mutter (10, 10') aufzunehmen und festzuhalten, wobei die Mutter (10, 10') in ihrer Mulde (9) im Verhältnis zu der Trägerplatte (1, 1') drehmäßig blockiert ist und die Mutter (10, 10') eine kugelförmige Kontaktfläche umfasst, die dazu geeignet ist, mit einer komplementären kugelförmigen Kontaktfläche zusammenzuwirken, die in ihrer Aufnahmemulde (9) eingerichtet ist, um ihr einen Freiheitsgrad in der Mulde (9) gemäß einem zulässigen vorherbestimmten Neigungsbereich zu verleihen, um eine Neigung ihrer Achse im Verhältnis zu der Achse der Aufnahmemulde (9) zu ermöglichen, wobei die Mutter (10, 10') dadurch eine mehrachsige Beschaffenheit aufweist, und dass mindestens eine der Kopfschrauben (17) einen Kopf (21) umfasst, der sich in einem Körper (22) verlängert, der mit einem Gewinde (23) zur Befestigung im Knochen und mit einem Gewinde (24), das dazu gedacht ist, mit dem Gewinde der dazugehörigen Plattenmutter (10) zusammenzuwirken, versehen ist.

4. Ausrüstung nach Anspruch 3, **dadurch gekennzeichnet, dass**:
- die erste Bohrbuchse (40) mit einem Gewindeansatz (42, 43) versehen ist, der dazu geeignet ist, um in eine der Muttern (10, 10') des Kopfteils (6, 6') eingeschraubt zu werden; und
- die zweite Bohrbuchse (32) dazu geeignet ist, um mit einer der Öffnungen (26) des Zielbügels (25, 25') zusammenzuwirken, der an einer der Muttern (10) des Kopfteils (6, 6') in Anschlag positioniert ist.

5. Ausrüstung nach einem der Ansprüche 1 bis 4, **dadurch gekennzeichnet, dass** die erste Bohrbuchse (40) einen Kopf (42) umfasst, der strukturiert und dimensioniert ist, um durch die Öffnungen (26) des Zielbügels (25, 25') gehen zu können, und zwar mit Spiel, so dass er über einen Freiheitsgrad gemäß einem Neigungsbereich verfügt, um das Umsetzen einer mehrachsigen Technik der dazugehörigen Kopfschraube durch die Öffnung (26) des Zielbügels (25, 25') an Ort und Stelle auf der Osteosynthese-Platte (1, 1') zu ermöglichen.

6. Ausrüstung nach einem der Ansprüche 1 bis 5, **dadurch gekennzeichnet, dass** sie einen Zielbügel (25, 25') umfasst, der mit einer einzigen durchgehenden Öffnung (27) für seine Befestigung an der Osteosynthese-Platte (1, 1') ausgestattet ist, und auf seiner Unterseite, die dazu gedacht ist, mit dem Kopfteil (6, 6') in Berührung zu kommen, mit mindestens einer Nase oder einer Aussparung versehen ist, die dazu gedacht ist, mit einher komplementären Nase oder Aussparung (13) zusammenzuwirken, die entsprechend auf der Oberseite (3) des Kopfteils (6, 6') eingerichtet ist.

7. Ausrüstung nach einem der Ansprüche 1 bis 6, **dadurch gekennzeichnet, dass** sie einen Satz von Positionierungsstiften (30) umfasst, und dass der Zielbügel (25, 25') und der Kopfteil (6, 6') entsprechende Öffnungen (14, 29) für den Durchgang der Positionierungsstifte (30) umfazsen,

8. Ausrüstung nach einem der Ansprüche 1 bis 7, **dadurch gekennzeichnet, dass** die zweite Bohrbuchse (32) eine Messskala (32') umfasst, die dazu gedacht ist, mit einer Markierung (31') zusammenzuwirken, die auf dem dazugehörigen Bohrer (31) angebracht ist, um die erreichte Bohrtiefe zu bestimmen, und um somit die zu verwendende geeignete Schraubenlänge (17) zu bestimmen.

9. Ausrüstung nach einem der Ansprüche 1 bis 8, **dadurch gekennzeichnet, dass** sie einen Messstab (34) umfasst, der dazu geeignet ist, die Länge des durchgehenden Vorbohrlochs zu bestimmen, das durch den angebohrten Knochen ausgebildet ist, wobei der Messstab (34) einen hohlzylindrischen Körper (35) umfasst, dessen Absatz (36) dazu geeignet ist, um sich auf der Osteosynthese-Platte (1, 1') abzustützen, gegebenenfalls durch eine der Öffnungen (26) des Zielbügels (25, 25') hindurch, der zu einer Gleitführung (37) gehört, deren eines Ende mit einer Messskala (38) versehen ist und deren anderes Ende mit einem Positionierungshaken (39) versehen ist.
